# EUROPEAN PATENT APPLICATION

(11) **EP 2 762 884 A1**
(43) Date of publication of application: **06.08.2014**
(21) Application number: 12836741.4
(22) Date of filing: 20.06.2012
(51) Int. Cl.: G01N 33/53, G01N 21/27, G01N 33/543

(54) **MOLECULAR TEMPLATE AND METHOD FOR PRODUCING SAME**

(30) Priority: 30.09.2011 JP 2011217179
(71) Applicant: Hitachi, Ltd., Tokyo 100-8280 (JP)
(72) Inventor: TAKEUCHI Toshifumi, Kobe-shi Hyogo 657-8501 (JP); TANIGUCHI Shinichi, Yokohama-shi Kanagawa 244-0817 (JP); AKAMATSU Naotoshi, Yokohama-shi Kanagawa 244-0817 (JP)
(74) Representative: Denison, Christopher Marcus
(86) International application number: PCT/JP2012/065727
(87) International publication number: WO 2013/046826

(57) **Abstract**

The method and apparatus for detecting a chemical substance of the present invention perform the detection of a chemical substance by capturing the chemical substance with a capturing body prepared by utilizing a molecular template. The present invention provides a chemical sensor easy to use for general consumers at home as well as for medical personnel (medical doctors, medical technicians and nurses). In particular, an object of the present invention is to diagnose the symptom of stress disorder by detecting, with high sensitivity, a steroid hormone such as cortisol closely related to stress disorder, and to herewith contribute to the prevention and early treatment of stress disorder. The present invention provides a molecular template for a steroid hormone, including a polymer to interact with the steroid hormone. The polymer preferably includes, in the polymerization unit thereof, two or more functional groups to interact with the steroid hormone.

## Description

### Technical Field

The present invention relates to a molecular template and a method for producing the same, and an apparatus for detecting a chemical substance and a method for detecting a chemical substance using the molecular template.

### Background Art

Chemical substances to be managed in the fields of, for example, clinical laboratory tests, environment, sanitation and disaster prevention diverge into many branches, and such chemical substances are extremely diverse in types. Examples of such chemical substances include: a hormone molecule to serve as a stress disorder marker, the endocrine disruptors in the problem of environmental hormones, the soil pollution substances in the sites of demolished factories, the asbestos released from construction materials, and the chemical substances causing stench or unpleasant taste generated from food and containers thereof, or apparatuses producing food and containers. Most of such chemical substances are low-molecular-weight molecules, and usually contained in extremely small amounts in the measurement objects. However, a rapid and high-sensitivity detection of such chemical substances is an extremely important operation for the purpose of ensuring the safety or the like in various fields.

Current measurement techniques come to enable the analysis of various chemical substances even at a ppt (one trillionth) or less level as a result of the combination of, for example, highly sophisticated separation techniques, concentration techniques and analytical methods. The case of such a trace level analysis is usually required to undergo various steps such as optimal separation, concentration, qualitative analysis and quantitative analysis, selected so as to be compatible with the detection object. Consequently, such a trace level analysis requires a great deal of labor and a long period of time, and a high analysis cost. Accordingly, an analytical method requiring such a large number of complicated steps is to be specialized as a measurement method in a laboratory, and is not suitable as a method to be used in measurement sites.

The measurement method required in measurement sites is a measurement method capable of detecting a chemical substance on the site. The sensor technique has developed techniques different from an analysis technique on the basis of such needs. A sensor method enables a simple and rapid detection or monitoring of chemical substances, and additionally is capable of miniaturizing measurement apparatuses.

On the other hand, the current sensor technique has not yet succeeded in achieving a molecular composition analysis with such a high sensitivity as realized in the analysis technique. However, in general, the detection of the chemical substances to be detection objects in the foregoing fields starts from the situation in which even the presence of such chemical substances in the measurement objects is not clear. Alternatively, even if such chemical substances are present, the amounts of such chemical substances are extremely small. Accordingly, a combination of concentration and separation are indispensable in the measurement; however, the measurement method undergoing such steps is an analytical method in a laboratory, and as described above, is not compatible with the method in measurement sites. There is also a problem such that as described above, the analysis ability of the current sensor technique cannot technically deal with the analysis of such chemical substances as described above.

The present inventors pay attention to a molecular template technique for the purpose of solving the foregoing problem. Specifically, the present inventors pay attention to the development of a sensor technique to detect a target chemical substance by selectively capturing the chemical substance, without requiring the steps of concentration and separation.

As the background art of the present technical field, Patent Literature 1 is quoted. This literature states that "devices, methods and kits for rapid and simple qualitative determination of target molecules including small molecules, polypeptides, proteins, cells and infectious disease agents in liquid samples are capable of performing real-time measurement of these entities in liquid samples, and are highly selective, highly sensitive, simple in operability, low in cost and portable; the devices, methods and kits provide, in at least some examples, the use of MIPs in a flow through or lateral flow device"(see the abstract). The MIP as described in this literature means a molecular template polymer (molecularly imprinted polymer), and the methods for synthesizing MIPs according to the chemical substances to be captured are widely known.

### Citation List

### Patent Literature

Patent Literature 1: WO2009/083975

### Summary of Invention

### Technical Problem

The technical problem of the present invention is to provide a molecular template for capturing a chemical substance, a detection object, and a method for producing the molecular template, and a method and an apparatus for detecting a chemical substance, identifying the chemical substance by using the molecular template rapidly at a high sensitivity with a low cost. Another technical problem of the present invention is to provide a method and an apparatus for detecting a chemical substance, capable of detecting the chemical substance, the detection object, at an ultrahigh sensitivity.

In other words, the method and apparatus for detecting a chemical substance of the present invention performs the detection of a chemical substance by capturing the chemical substance with a capturing body prepared by utilizing a molecular template. The present invention provides a chemical sensor easy to use for general consumers at home as well as for medical personnel (medical doctors, medical technicians and nurses). In particular, an object of the present invention is to early diagnose the symptom of stress disorder by detecting with high sensitivity a steroid hormone such as cortisol closely related to stress disorder, and to herewith contribute to the prevention and early treatment of stress disorder.

### Solution to Problem

For the purpose of rapidly, inexpensively and highly sensitively detecting a steroid hormone such as cortisol, the present invention solves the foregoing technical problem by synthesizing a molecular template polymer (MIP) appropriate for a steroid hormone. Specifically, for example, the present invention adopts the constitution described in the claims. The present application includes a plurality of means for solving the foregoing technical problem; as an example to be quoted of such means, the molecular template according to the present invention is "a molecular template for a steroid hormone, wherein the molecular template includes a polymer to interact with the steroid hormone."

Although the synthesis principle of MIP has been known since 1950s, it is necessary to elaborately investigate the synthetic raw materials, synthetic route, reaction time and reaction temperature appropriate for a chemical substance (target) to be captured. Thus, it is possible to propose as a principle the device structure utilizing such MIP as quoted in foregoing Patent Literature 1; however, for the purpose of practically preparing a MIP to capture a target with a high sensitivity, an elaborate design, an elaborate synthesis and an elaborate purification come to be necessary.

A molecular template prepared by molecular imprinting can be constructed by using various matrices. The present inventors have discovered a polymer to be used in molecular imprinting for the molecule of a steroid hormone such as cortisol closely related to stress disorder or the derivatives of cortisol.

The polymer in the present invention is appropriate as the matrix to be used for imprinting of a steroid hormone in that the network structure of the polymer has an appropriate flexibility, and is swollen or contracted according to a factor such as a solvent or an environment. In other words, the recognition site formed by the template molecule in the template polymer is required to be close in size to the template molecule. On the other hand, for the purpose of removing the template molecule after the polymerization or allowing the chemical substance (target) to be again bonded to the recognition site, a space large to some extent is required so as to allow the molecule to migrate in the network structure. The present inventors have discovered a polymer material satisfying such conflicting conditions and the synthesis conditions of the polymer material. In particular, a steroid hormone such as cortisol has a steroid skeleton, and hence the molecule thereof is rigid; and a steroid hormone such as cortisol has groups such as hydroxyl groups, and hence are capable of forming interactions with the raw material monomer, required at the time of molecular imprinting. In particular, in the present invention, by using a dicarboxylic acid derivative capable of interacting with, for example, cortisol at two sites, as a fraction of the raw material monomer, a synthesis of a molecular template enabling a highly efficient capture is achieved.

The method for detecting a chemical substance of the present invention acquires a highly sensitive detection capability by constituting the method so as to enhance the sensitivity of detection of the captured steroid hormone.

The present description includes the contents described in the specification and the drawings of Japanese Patent Application No. 2011-217179, which is a basis of the priority of the present application.

### Advantageous Effects of Invention

According to the method and apparatus for detecting a chemical substance of the present invention, it is possible to selectively detect a steroid hormone to be detected owing to the molecular template formed of a specific polymer, without needing any concentration step or any separation step.

According to the apparatus for detecting a chemical substance of the present invention, it is possible to miniaturize the molecule capturing section corresponding to the most important sensor portion, and hence it is possible to provide a portable apparatus for detecting a chemical substance.

### Brief Description of Drawings

[Figure 1] Figure 1 is a diagram schematically illustrating a method for producing a molecular template.
[Figure 2] Figure 2 is a conceptual diagram for illustrating the constitution of an apparatus for detecting a chemical substance.
[Figure 3] Figure 3 is a diagram showing a synthesis scheme of a molecular template.
[Figure 4A] Figure 4A is a diagram showing the molecular structure of cortisol.
[Figure 4B] Figure 4B is a diagram showing the molecular structure of itaconic acid.
[Figure 5] Figure 5 is a diagram schematically illustrating the interaction between cortisol and itaconic acid.
[Figure 6] Figure 6 is a diagram showing the molecular structures of different steroid hormones.
[Figure 7] Figure 7 is a conceptual diagram illustrating a competition method.
[Figure 8] Figure 8 is a diagram illustrating an embodiment of the apparatus for detecting a chemical substance.
[Figure 9] Figure 9 is a graph showing a calibration line of the concentration of cortisol.
[Figure 10] Figure 10 is a graph showing the detection results of cortisol.
[Figure 11] Figure 11 is a diagram showing the molecular structure of methacryloylated cortisol.
[Figure 12] Figure 12 is a diagram showing the molecular structure of progesterone.
[Figure 13] Figure 14 is a graph showing a calibration line of the concentration of progesterone.
[Figure 14] Figure 14 is a graph showing the detection results of cortisol and progesterone.

### Description of Embodiments

Hereinafter, the embodiments of the present invention are described. The present invention is not limited to these embodiments at all, and can be implemented in various modes within a range not departing from the gist of the present invention.

An embodiment of the apparatus for detecting a chemical substance of the present invention is constituted with a molecule capturing section having on the surface thereof a capturing body including a molecular template formed by utilizing a specific chemical substance, and a captured amount measurement section for quantitatively determining the chemical substance captured in the molecule capturing section. The capturing body can capture the specific chemical substance (target) in a specimen in a manner depending on the specific molecular structure possessed by the chemical substance. The apparatus for detecting a chemical substance of the present embodiment performs molecular recognition of a chemical substance on the basis of this technique.

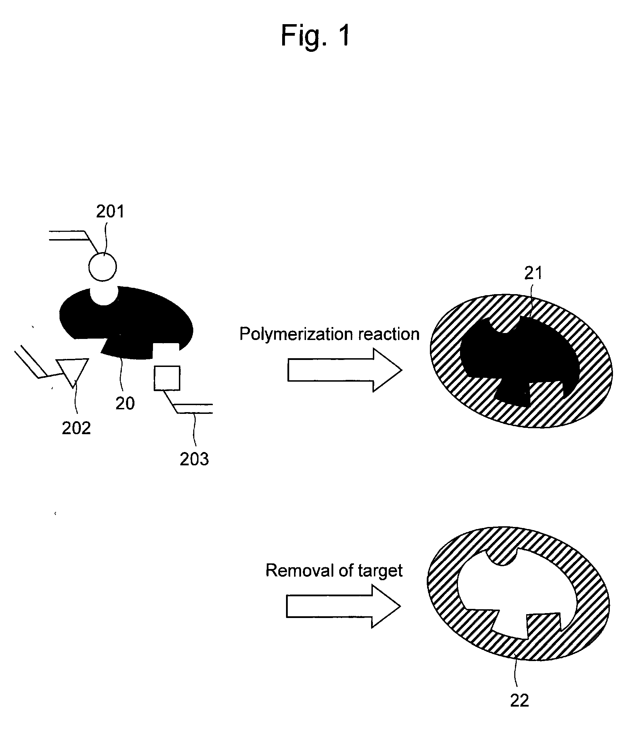

Figure 1 shows the preparation principle of a molecular template (MIP) 22 in the present embodiment. First, a polymerization reaction is performed in a mixture composed of a target 20 to be captured, and a monomer raw material A 201, a monomer raw material B 202 and a monomer raw material C 203 to interact with the target 20, and thus a recognition site 21 of the target 20 is formed. Subsequently, the target 20 is removed by an operation such as washing, and thus the molecular template (MIP) 22 having a recognition site 21 can be prepared. Here is shown an example in which the target 20 is used as the template molecule for forming the recognition site 21; however, in place of the target 20, the derivatives and analogs of the target 20 may also be used.

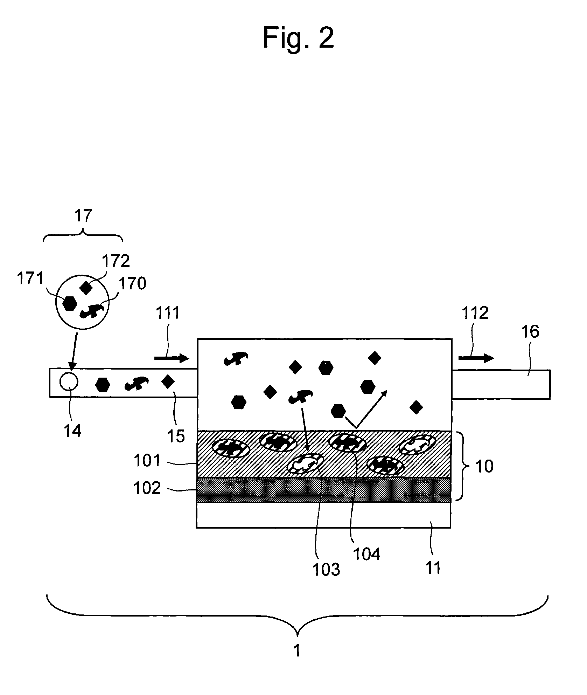

Figure 2 is a conceptual diagram for illustrating the constitution of the apparatus for detecting a chemical substance according to the present embodiment. As shown in Figure 2, the apparatus 1 for detecting a chemical substance according to the present embodiment includes a molecule capturing section 10, a captured amount measurement section 11, a sample injection section 14, a sample conveyance section 15 and a discharge section 16. Hereinafter, the individual constitutions are described in detail.

Into the sample injection section 14, a specimen 17 is injected. The specimen 17 includes a target 170, which is an object to be detected, a foreign substance A 171 and a foreign substance B 172. Needless to say, some specimens include no target 170, or a larger number of types of foreign substances. The specimen 17 is conveyed in the direction of an arrow 111 or an arrow 112.

The molecule capturing section 10 is constituted with a capturing body 101 and a support 102. The capturing body 101 includes a molecular template 103 or a molecular template 104 capturing the target. The capturing body 101 is disposed on the surface of the molecule capturing section 10, and is mainly composed of a molecular template (MIP). The support 102 supports the capturing body 101, and is a solid constituting the main shape of the molecule capturing section 10. The material of the support 102 is not particularly limited, as long as the material can maintain a predetermined shape. Specific examples of the material of the support 102 include: plastic, metal, glass, synthetic rubber, ceramic, water-proofed or reinforced paper, or combinations of these. In the molecule capturing section 10, the surface having the capturing body 101 may be a surface covering the whole or a portion of the molecule capturing section 10.

The molecule capturing section 10 can be formed by combining the separately and independently prepared capturing body 101 and support 102. The support 102 may be constituted with a multilayer structure composed of different constituent components. For example, the case where the support 102 is composed of two layers, namely, a glass substrate and a gold (Au) thin film is relevant. The method for combining the capturing body 101 and the support 102 is not particularly limited, as long as the molecule capturing section 10 is constituted in such a way that the target capturing information can be output to the below-described captured amount measurement section 11. For example, the capturing body 101 and the support 102 may be directly combined with each other, or may be combined through the intermediary of one or more other connection substances to connect these two members. The molecule capturing section 10 may also be constituted by integrating the capturing body 101 and support 102 made of one and the same material. For example, a case where a polymer itself including a molecular template doubles as the support is relevant.

The molecule capturing section 10 is constituted at least in such a way that the surface having the capturing body 101 can be directly brought into contact with the specimen 17. This is because the capturing body 101 is allowed to capture the target 170 to be detected. Here, the "specimen" means a liquid or a solid to be a measurement object.

The capturing means the capturing through bonding or interaction. The capturing is a concept including both direct capturing and indirect capturing. For example, the capturing may be a direct capturing of the target 170 to be detected, by the capturing body 101 of the molecule capturing section 10, or an indirect capturing of the target to be detected, through the intermediary of a second capturing body fixed on the molecule capturing section.

The capturing body 101 includes the molecular template formed by using a specific template molecule, and can capture the chemical substance to be a target in a manner depending on the specific molecular structure possessed by the target. The material of the capturing body 101 is not particularly limited as long as the material has a function to capture the target in a manner depending on the specific molecular structure. For example, the material may be a protein, a polymer or a metal. Specifically, for example, antibodies and molecular templates are relevant.

The method for producing the molecular template used in the present invention may be based on the molecular imprinting method generally used, for example, in the field of sensors. For example, first, in the presence of a chemical substance to be a target, a functional monomer to interact with the target through ionic bonding or hydrogen bonding is copolymerized with other monomer components used if necessary, and thus, the target is fixed in the polymer. In this case, the copolymerization ratio between the functional monomer and the other monomer components is varied depending on, for example, the types of the individual monomer components and not particularly limited; however, for example, it is possible to set such that the functional monomer:the other monomer components = 1:16 to 1:64 (molar ratio). In particular, it is desirable that this ratio is 1:32. Subsequently, the target is removed from the polymer by washing. The cavity (space) left in the polymer memorizes the shape of the target, and is provided with a chemical recognition ability due to the functional monomer fixed in the cavity.

In the present embodiment, as the target, an example of a steroid hormone is described; however, examples of the target include, without being limited to steroid hormones, various substances being present, under normal temperature and normal pressure, in a state of being vaporized or in a state of being liquid (inclusive of, for example, the cases of being dissolved in a solvent). For example, volatile chemical substances, electrolytes, acids, bases, carbohydrates, lipids and proteins are relevant. Examples of the target also include the chemical substances capable of being present only in a state of being a solid under normal temperature and normal pressure, and capable of being present as fine particles in gases or in liquids. Those targets which have corrosion effects, dissolution effects, modification effects on the molecule capturing section are unsuitable.

The molecular weight of the target is not particularly limited as long as the molecular weight allows the capturing body 101 to capture the target; however, in the present invention taking as its main object the detection of low-molecular-weight chemical substances, the target is preferably a low-molecular-weight molecule having a molecular weight of the order of a few tens to a few hundreds.

The molecule capturing section 10 may be constituted in a manner detachable from the apparatus 1 for detecting a chemical substance with the aid of a detachable part. This is for the purpose of enabling the selection of an optimal molecule capturing section according to, for example, the measurement environment or the state of the specimen, or for the purpose of omitting the labor and time for washing a once used molecule capturing section, and additionally, for the purpose of precluding the risk of contamination due to continuous use. The molecule capturing section detached with the aid of the detachable part is not necessarily required to be the whole of the molecule capturing section, but may be a portion having the capturing body 101.

The detachable part may have, for example, a fixing member for fixing the molecule capturing section 10 to the apparatus 1 for detecting a chemical substance, and a terminal for transmitting information to and receiving information from the molecule capturing section 10. In the case where an apparatus 1 for detecting a chemical substance has a plurality of molecule capturing sections 10, there may be a plurality of detachable parts.

The captured amount measurement section 11 is constituted so as to be capable of quantitatively determining the chemical substance captured in the molecule capturing section 10. The phrase of the "quantitative determination of a chemical substance" means the measurement of the amount of the molecules of the chemical substance to be the target, captured by the capturing body 101 when the specimen 17 is exposed to the molecule capturing section 10 for a predetermined period of time. The "predetermined period of time" as referred to herein means an optional period of time beforehand determined before the quantitative determination. For example, the predetermined period of time may be 1 second or 1 minute. In the quantitative determination, the dynamic change of the capturing body 101, when the capturing body 101 captures the target 170 present in the specimen 17, is transformed into an electric signal, and on the basis of, for example, the intensity of the electric signal, the captured target can be measured. The method for the quantitative determination is not particularly limited as long as the dynamic change of the capturing body 101 can be transformed into an electric signal. For example, a surface plasmon resonance measurement method, a quartz crystal microbalance measurement method, an electrochemical impedance method, a colorimetric method or a fluorescence method can be adopted. The quantitative determinations based on these methods can all perform the measurement in a period of time of 100 ms (0.1 second) or less.

The surface plasmon resonance measurement method is also referred to as the SPR (surface plasmon resonance) method, and is a method for measuring with a high sensitivity a trace amount of a captured object on a metal thin film by utilizing the surface plasmon resonance phenomenon such that with the variation of the incidence angle of a laser light beam incident on the metal thin film, the reflected light intensity is attenuated. Specifically, the particle-like solid obtained by grinding the molecular template of the present invention is suspended in a solvent (water, or an organic solvent), the resulting suspension is spin-coated on a metal thin film, dried and subjected to a measurement. In the measurement, the surface plasmon is generated on the surface side of the metal thin film. When the wave number of an evanescent wave and the wave number of a surface plasmon coincide with each other, the photon energy is used for exciting the surface plasmon through resonance, and hence a phenomenon of the attenuation of the reflected light is caused. This can be found as the attenuation of the reflected light intensity accompanying the variation of the incidence angle of the laser when the incidence angle of the laser is varied. The incidence angle (referred to as the resonance angle θ) when the reflected light intensity ratio which is the ratio of the reflected light intensity to the incident light intensity is affected by the interaction between substances occurring on the metal surface. Accordingly, the interaction between the substances can be observed as the variation of the resonance angle θ between before and after the interaction. For example, when the resonance angle in the state in which the molecular template supported on the metal thin film surface does not capture anything is represented by θ₀, the resonance angle is changed to θ₁ when the molecular template captures the target. In this case, by observing the value of Δθ as the difference between θ₁ and θ₀, the amount of the target captured by the molecular template can be quantitatively determined. Accordingly, for example, cortisol contained in the specimen in a concentration of 125 µM can be quantitatively determined. When spin coating is performed, it is important to form the film with the distance over which the plasmon resonance propagates. Specifically, it is preferable to form a film with a thickness of 100 nm.

The quartz crystal microbalance measurement method is also referred to as the QCM (quartz crystal microbalance) method, and is a mass measurement method of quantitative determination of an ultra-trace amount of a substance attached on a quartz crystal unit on the basis of the variation magnitude of the resonance frequency of the quartz crystal unit due to the attachment of the substance on the surface of the quartz crystal unit. Specifically, the particle-like solid obtained by grinding the molecular template of the present invention is suspended in a solvent (water, or an organic solvent), the resulting suspension is spin-coated on the sensor of a quartz crystal unit, dried and subjected to a measurement. The measurement method is a heretofore known established method, thus the measurement may be performed according to the conventional technique, and hence a detailed description of the measurement is omitted. For the purpose of achieving the measurement reproducibility, the thickness of the film formed on the quartz crystal unit is preferably set at 1 µm or less.

The electrochemical impedance method is also referred to as the surface-polarization controlling method, and is a method in which by controlling the surface polarization of a metal through the electrode potential, the interaction between the electrode surface and the substance attached to the electrode surface is varied, and thus the information about the attached substance is obtained. Specifically, the particle-like solid obtained by grinding the molecular template of the present invention is suspended in a solvent (water, or an organic solvent), the resulting suspension is spin-coated on the electrode surface, dried and subjected to a measurement. The measurement method is a heretofore known established method, thus the measurement may be performed according to the conventional technique, and hence a detailed description of the measurement is omitted. For the purpose of achieving the measurement reproducibility, the thickness of the film formed on the electrode surface is preferably set at 1 µm or less.

The colorimetric method and the fluorescence method are different from each other only in the nature of the substrate used for detection, and are almost the same each other in the principle involved. Specifically, in the case where the substrate produces a chromogenic substance, the method concerned is referred to as the colorimetric method; in the case where the substrate produces a fluorescent substance, the method concerned is referred to as the fluorescence method. In either of these methods, the substrate or the like as the probe for detection is supported on the capturing body, or on a mediator or the like, the color optical density or the fluorescence intensity based on the substrate is measured with an absorption spectrophotometer or a luminometer or the like, and thus, the bonding with the target is quantitatively determined. In the case where the capturing body is an antibody, these methods correspond to the ELISA method and the like. The ELISA method is also referred to as the enzyme linked immunosorbent assay method. The principle of the ELISA method is such that the primary antibody bonded to the target is made to produce a chromogenic substance or a fluorescent substance by the action of the enzyme concerned, for example, through the intermediary of the secondary antibody, which is an enzyme-labeled mediator, and the target is quantitatively determined on the basis of the color optical density of the chromogenic substance or the fluorescence intensity of the fluorescent substance. In the case of the molecular template, for example, a molecular template having in the cavity a functional monomer supporting a substrate probe is relevant. For example, the capturing of the target in the molecular template changes the state of the substrate probe in the cavity so as to develop a color or emit fluorescence, and the target can be quantitatively determined on the basis of the color optical density of the developed color or the fluorescence intensity of the emitted fluorescence.

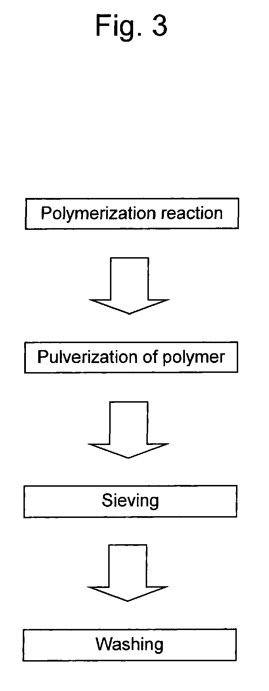

Figure 3 shows a synthesis scheme of the molecular template. After the polymerization reaction, the produced polymer is ground, and subsequently allowed to pass through the steps of sieving and washing, and thus the molecular template can be obtained. Hereinafter, a description is made on the case where according to this procedure, a molecular template of cortisol, which is a steroid hormone, is synthesized by using itaconic acid as a raw material.

### (Production of Molecular Template of Cortisol)

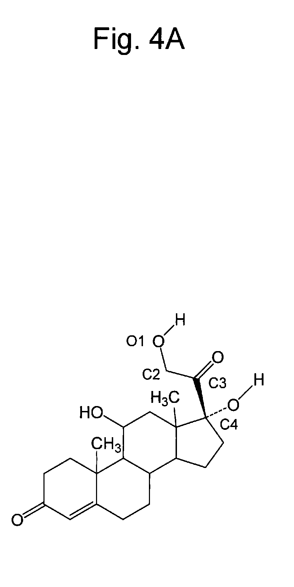

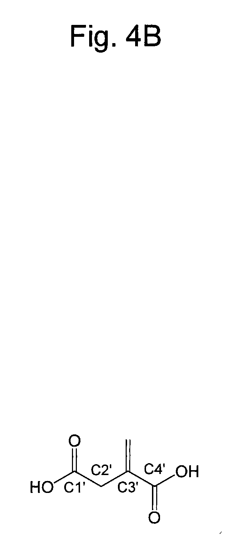

In the presence of cortisol, the polymerization of a functional monomer interacting with cortisol monomer is performed, and by washing the polymer obtained by the polymerization reaction, it is possible to obtain a molecular template, specifically recognizing cortisol, in the interior of the polymer. Figure 4A shows the molecular structure of cortisol. Figure 4B shows the molecular structure of itaconic acid. As shown in Figure 4A, the carbon atom in the terminal five-membered ring in the skeleton of cortisol is denoted by C4, and accordingly, the carbon atom next to C4 of the carbonyl group is denoted by C3, the carbon atom next to C3 of the methylene group is denoted by C2, and the oxygen atom next to C2 of the hydroxyl group is denoted by O1. When the skeleton is assumed to extend to O1, the bonds are formed from the terminal of the steroid skeleton, through two carbon atoms, to the oxygen atom.

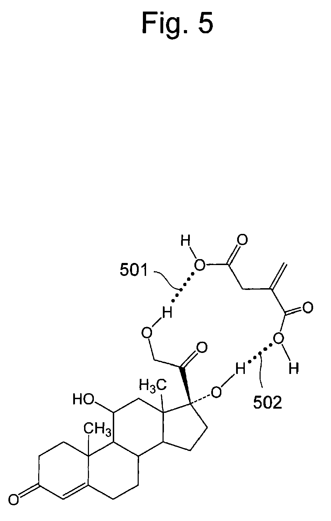

On the other hand, in itaconic acid as shown in Figure 4B, the carbon atom of the carboxyl group on the left side of the figure is denoted by C1', and accordingly, the carbon atom next to C1' of the methylene group is denoted by C2', and the carbon atom next to C2' of the vinyl group is denoted by C3', and the carbon atom next to C3' of the carboxyl group is denoted by C4'. The important point in the production of the molecular template of the present invention is the strength of the interaction force between the target and the polymerizable monomer. The use of itaconic acid as the raw material for the molecular template of cortisol is based on the observation that carboxyl groups are located on both terminals of the itaconic acid molecule, the distance between these groups is appropriate, and accordingly, itaconic acid may be assumed to be easy to interact with a moiety of cortisol. Figure 5 schematically illustrates the interaction between cortisol and itaconic acid. As the dotted line 501 and the dotted line 502 indicate, the use of itaconic acid allows the interaction with cortisol to occur at a plurality of sites. In this way, the inclusion, in the polymerization units, of a monomer having two or more functional groups to interact with a steroid hormone such as cortisol improves the fitting property between the steroid hormone and the monomer, and thus a significant property as the molecular template is considered to be able to be brought about.

The "functional group" as referred to herein means an atomic group included in a group of chemical substances and exhibiting common chemical properties and reactivities in the group of chemical substances. Examples of the functional group include: a hydroxyl group, an aldehyde group, a carboxyl group, a carbonyl group, a nitro group, an amino group, a sulfone group and an azo group. When the monomer to interact with a steroid hormone has two or more functional groups, the functional groups are particularly preferably carbonyl groups.

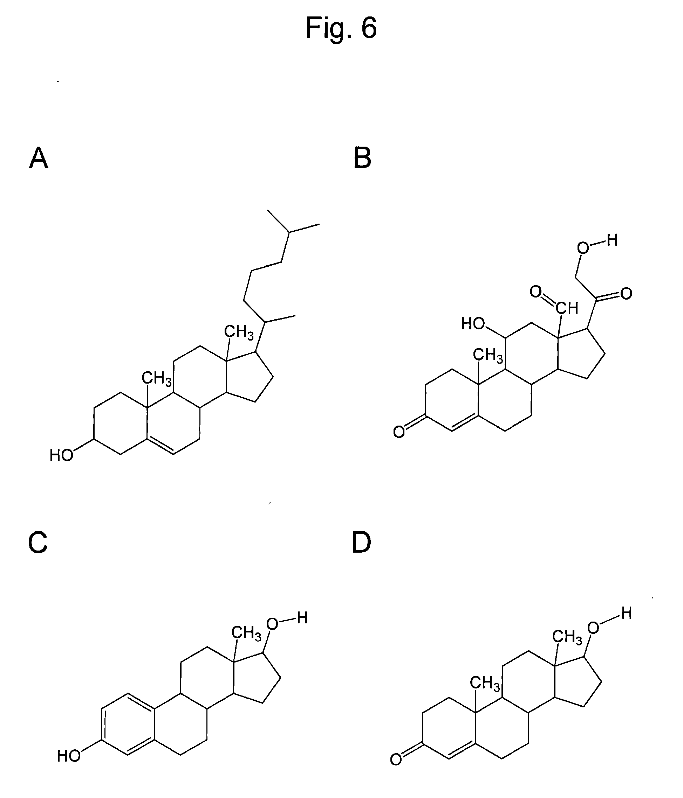

For steroid hormones other than cortisol, by utilizing polymerizable monomers to interact preferably at a plurality of sites, molecular templates can be synthesized. Natural steroid hormones are generally synthesized in gonads and adrenal glands from cholesterol. Figure 6 shows the molecular structures of cholesterol and representative steroid hormones. When the foregoing method for synthesizing the molecular template of cortisol is used, molecular templates for other steroid hormones can be prepared. A of Figure 6 shows cholesterol, and aldosterone of B of Figure 6, estradiol of C of Figure 6 and testosterone of D of Figure 6 are metabolically synthesized with cholesterol as mother skeleton.

As described above, itaconic acid is preferably used as the raw material for the molecular template for cortisol, as a result of selecting the raw material aiming at multiple site formation of hydrogen bonds. Similarly to this, it is possible to select a monomer structure suitable for a steroid hormone having a steroid skeleton high in planarity. For aldosterone of B of Figure 6, a monomer raw material for a molecular template can be selected by paying attention to the two items, namely, the hydroxyl group (OH) present at a terminal through the intermediary of a carbonyl group and a methylene group, and the aldehyde group (CHO) directly bonded to the skeleton. It is recommended to use, as a raw material for the molecular template, a monomer molecule having a length allowing simultaneously interacting with such a plurality of functional groups as described above. Specifically, it is recommended to produce a molecular template by using as a polymerization unit a monomer which is a vinyl monomer, has two carboxyl groups in the skeleton thereof, and has a distance (a distance of two or three in terms of methylene group) appropriate for fitting to the target of the molecular template. Similarly to the molecular template of cortisol, in the presence of the steroid hormone to be the target, the foregoing vinyl monomer and other monomer components such as styrene and divinylbenzene are copolymerized if necessary together with a polymerization initiator, and thus, a molecular template can be obtained. Instead of performing copolymerization, the vinyl monomer to undergo the interaction may also be homopolymerized. When the foregoing vinyl monomer and other monomer components are copolymerized with each other, the copolymerization ratio between these monomers is varied depending on the individual monomer components and the type and the like of the steroid hormone, and is not particularly limited; however, the copolymerization ratio between these monomers can be specified for example as follows: vinyl monomer to interact with steroid hormone:other monomer components = 1:16 to 1:64 (molar ratio). The foregoing ratio is particularly preferably 1:32.

Estradiol of C of Figure 6 and testosterone of D of Figurer 6 each have functional groups distant from each other, and accordingly at the time of producing a molecular template, one monomer is not required to simultaneously interact at a plurality of sites; it is recommended to perform copolymerization, by using a plurality of polymerizable monomers respectively recognizing the functional groups, in the presence of the target, together with styrene, divinylbenzene, a polymerization initiator and the like.

In the foregoing example, a description is made on the case where the interaction due to hydrogen bonding is formed between a steroid hormone and a monomer; however, as another embodiment, the steroid hormone to be a template molecule is converted into a derivative, and into the derivative, functional groups to undergo copolymerization reaction with the monomer to form the molecular template may also be introduced. The formation of the covalent bonds between the steroid hormone and the monomer due to the copolymerization reaction more strengthens the interaction between the steroid hormone and the monomer, improves the fitting property between the steroid hormone and the monomer, and can provide the advantageous properties as the molecular template. As the monomer to be copolymerized with the steroid hormone, similarly to the foregoing, monomers having two or more functional groups such as itaconic acid and a plurality of types of monomers can be used in combination.

Examples of the functional groups to be introduced into the steroid hormone molecule and to be copolymerized with the monomer include: polymerizable substituents such as an acryloyl group, a methacryloyl group, a vinyl group and an epoxy group; among these, in particular, a methacryloyl group is preferable.

The molecule capturing section may be constituted so as to enhance the sensitivity of detection of a steroid hormone with the aid of the competition method or the substitution method. The "substitution method" is a method utilizing the competition with respect to the capturing body, occurring between a chemical substance having a specific molecular structure beforehand captured by the capturing body and the target to be detected in the specimen. For example, when the capturing body is an antibody, the antibody is fixed on a support, and a composite antigen having a specific molecular structure is captured by the antibody. In this state, when the specimen including the target to be detected is exposed to the molecule capturing section, the composite antigen is dissociated from the antibody and the target to be detected in the specimen is captured, instead of the composite antibody, by the antibody, due to the difference in bonding strength. By quantitatively determining the change due to the substitution reaction, the target can be quantitatively determined with a high sensitivity. For example, when the surface plasmon resonance measurement method is used, the change of the resonance angle θ due to the substitution reaction may be observed. The enhancement of the detection sensitivity due to the substitution method allows even the target having a concentration of ppt level to be detected.

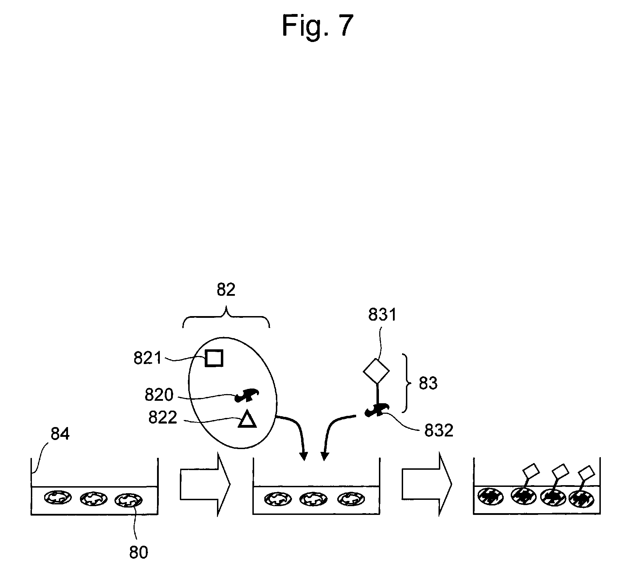

An example using the competition method is described on the basis of Figure 7. As shown in Figure 7, in a container 84, an aqueous suspension of a molecular template 80 is placed, and a solid or an aqueous solution of a specimen 82 and a solid or an aqueous solution of a labeled target 83 are placed in the container 84. The specimen 82 includes a target 820, a foreign substance A 821 and a foreign substance B 822. Needless to say, there possibly occurs a case where the target 820 is absent, or a case where a large number of types of foreign substances are present. The labeled target 83 is composed of the target moiety 832 and the label moiety 831. The target 820 and the labeled target 83 are allowed to react with the molecular template 80 for 1 hour at room temperature while the target 820 and the labeled target 83 are being allowed to compete against each other, and then the colorimetric magnitude or the fluorescence magnitude of the label moiety 831 is measured; and thus, the target amount in the specimen 82 can be derived. In other words, the larger the target amount, the smaller the fluorescence intensity or the paler the color of the solution. For the derivation of the target amount in the specimen 82, a separately derived colorimetric or fluorescence calibration line may be used. From this measurement, for example, cortisol contained in the specimen in a concentration of 125 µM or less can be quantitatively determined in a dominant manner.

In the foregoing embodiment, the electric signal acquired in the captured amount measurement section is frequently feeble in usual cases, and hence the acquired electric signal may be amplified if necessary. The amplification can be performed by means such as installation of an amplifier in the captured amount measurement section. When the acquired electric signal is an analog signal, the analog signal may be subjected to AD conversion if necessary. The AD conversion can be performed by means such as installation of an AD converter such as a comparator in the captured amount measurement section.

The captured amount measurement section is constituted so as to be able to output the measurement results. The destination of the measurement results is not particularly limited. For example, the measurement results may be output to an external display device such as a monitor. When the measurement results are output, the output form is not particularly limited. The output may be an output through direct wiring, or an output through a cable with a provided connection terminal such as a USB terminal. Alternatively, the measurement results may also be wirelessly transmitted.

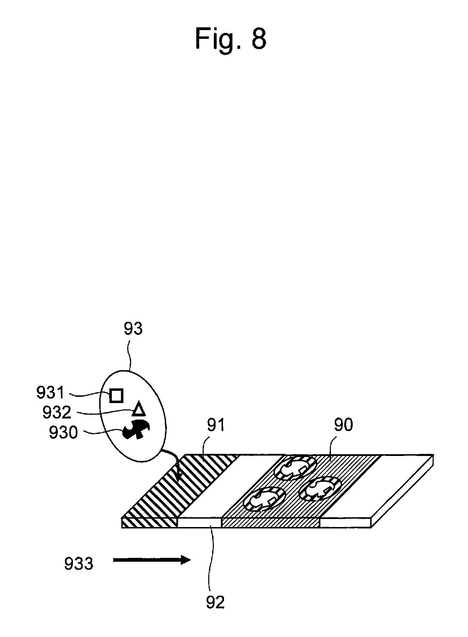

Figure 8 shows another embodiment of the apparatus for detecting a chemical substance of the present invention. The apparatus for detecting a chemical substance of Figure 8 is prepared by applying a molecular template to a material mainly such as a resin, glass, silica gel, paper or a metal. The detection apparatus is broadly composed of three parts, namely, a sample injection section 91, a capture/detection section 90, and a pretreatment layer 92. In the pretreatment layer 92, a non-woven fabric to adsorb proteins, lipids and the like in saliva is fixed. Accordingly, proteins, lipids and the like to disturb the detection of a steroid hormone such as cortisol are made not to enter the capture/detection section 90. The material used for the pretreatment layer 92 is not limited to a non-woven fabric, and may be a material such as a resin, glass, silica gel or paper. To the capture/detection section 90, the molecular template is applied. When the substitution method is utilized, a predetermined amount of a labeled steroid hormone may be beforehand immobilized. Next, a specimen 93 is applied to the sample injection section 91. The specimen 93 includes a target 930, a foreign substance A 931 and a foreign substance B 932. When the competition method is utilized, a labeled target is mixed in the specimen 93 and the resulting mixture is applied to the sample injection section 91. Subsequently, the specimen 93 and the labeled target travel in the direction of an arrow 933, and in the pretreatment layer 92, a fraction or the whole of the foreign substances in the specimen 93 is removed. Then, the target 930 and the labeled target in the specimen 93 are captured by the molecular template of the capture/detection section 90. By using for detection a fluorescence microscope, visual verification, an optical microscope or the like, the detection can be performed by evaluating the developed color or the like. By using this chip-shaped apparatus for detecting a chemical substance, for example, a target of a concentration of 125 µM or less in the specimen can be detected.

### Examples

Next, the present invention is described in more detail on the basis of Examples. However, following Examples are presented only for exemplification of the present invention, and the present invention is not limited by these Examples at all.

### (Example 1)

By using THF (tetrahydrofuran) as a solvent, a synthesis of a molecular template was performed by the following method. First, to a mixture of cortisol:itaconic acid:styrene:divinylbenzene (DVB) = 1:4:4:20 (molar ratio), V-70 (2,2'-azobis(4-methoxy-2,4-dimethylvaleronitrile)), a polymerization initiator, was added in a proportion of 2% by mole in relation to the monomer component.

Specifically, according to the recipe in Table 1, the polymerization of the molecular template polymer (MIP) was performed. In a vial, 362.5 mg (1 mmol) of cortisol, 524 mg (4 mmol) of itaconic acid, 418 mg (4 mmol) of styrene and 2604 mg (20 mmol) of divinylbenzene (DVB) were added and dissolved in THF (20 ml); then the resulting solution was transferred into a φ18 x 180 mm test tube, and 2,2'-azobis(4-methoxy-2,4-dimethylvaleronitrile) (V-70) (0.56 mmol), a polymerization initiator, was dissolved in the solution. The test tube was sealed with a septum cap and the air in the test tube was replaced with nitrogen, and then a polymerization was performed in a water bath set at 30°C for 72 hours.

For a reference experiment, a polymerization of a non-molecular template polymer (NIP: non-imprinted polymer) was performed. According to the recipe in Table 1, in a vial, without adding cortisol, 524 mg (4 mmol) of itaconic acid, 418 mg (4 mmol) of styrene and 2604 mg (20 mmol) of divinylbenzene (DVB) were dissolved in THF (20 ml); then, the resulting solution was transferred into a φ18 x 180 mm test tube, and 2,2'-azobis(4-methoxy-2,4-dimethylvaleronitrile) (V-70) (0.56 mmol), a polymerization initiator, was dissolved in the solution. The test tube was sealed with a septum cap and the air in the test tube was replaced with nitrogen, and then a polymerization was performed in a water bath set at 30°C for 72 hours.

**[Table 1]**

| Raw materials | MIP | NIP |
|---|---|---|
| Cortisol | 362.5 mg (1 mmol) | None |
| Itaconic acid | 524 mg (4 mmol) | 524 mg (4 mmol) |
| Styrene | 418 mg (4 mmol) | 418 mg (4 mmol) |
| DVB | 2604 mg (20 mmol) | 2604 mg (20 mmol) |
| V-70 | 172.7 mg (0.56 mmol) | 172.7 mg (0.56 mmol) |
| THF | 20 ml | 20 ml |

The polymer after the polymerization was dried under reduced pressure, ground with a mortar, then washed by using a Soxhlet extractor with acetone, methanol/acetic acid (9/1, volume/volume) and methanol, in this order; the washing was performed until cortisol, the target, was not able to be detected from the solvents. The non-molecular template polymer (NIP) containing no target was also polymerized and washed in the same manner as described above.

### • Evaluation of Cortisol Molecular Template Polymer

An adsorption experiment was performed by using the polymer after washing. The acetonitrile solutions of cortisol regulated to the concentrations of 0, 125, 250, 500 and 1000 µM, respectively, were prepared, and 1 mL of each of the solutions was measured with a pipette and placed in a microtube. Then, 5 mg of MIP or NIP was weighed out and added in each of the microtubes, and each of the microtubes was capped, stirred at 25°C and 800 rpm, and subjected to an incubation for 40 hours. After the incubation, the polymer was removed by filtration, and the cortisol in the solution was quantitatively determined by HPLC. The membrane used for the filtration allows a solid of 50 µm or less to pass therethrough. From the results of the quantitative determination, the amounts adsorbed to the molecular template polymer in the respective concentrations were determined.

The HPLC conditions were as follows: for the column, CHROMOLITH (registered trademark) RP-18e 100-4.6 manufactured by Merck KGaA was used; for the eluent, acetonitrile/H₂O (70/30, volume/volume) was used; the flow rate was set at 1mL/min; and for detection, UV light (241 nm) was used.

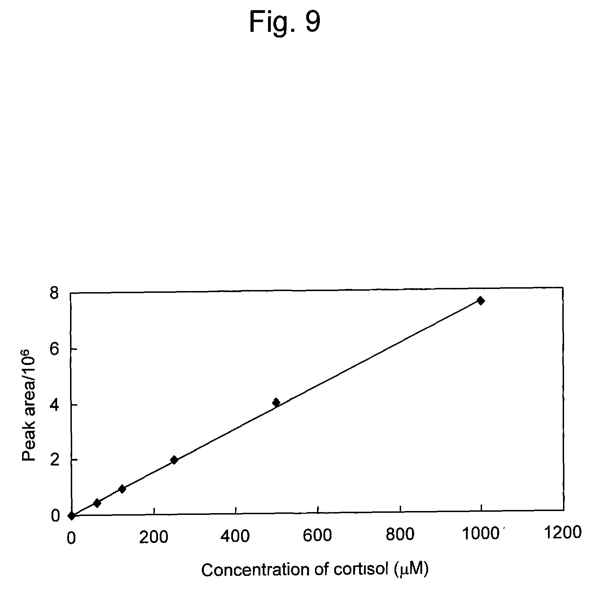

First, before the adsorbed amount was determined, a HPLC calibration line was prepared. Figure 9 shows the prepared calibration line. The abscissa axis of the graph indicates the concentration of cortisol (µM), and the ordinate axis indicates (1/10⁶) the peak area determined from HPLC. The samples respectively having the concentrations of cortisol of 0, 62.5, 125, 250, 500 and 1000 µM were used, and the peak areas for the respective concentrations were plotted against the respective concentrations. The trendline of this plot was found to be represented by Y = 7589.7X, and the squared correlation coefficient (R²) was found to be 0.99. By using this calibration line, the amounts adsorbed to MIP and NIP were derived.

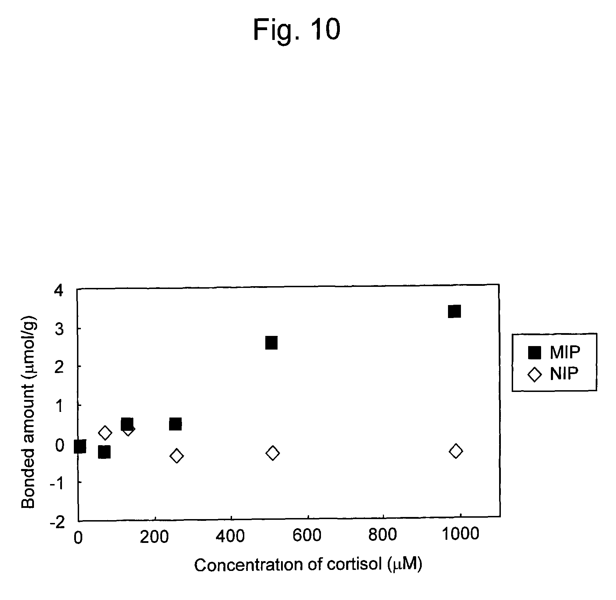

The concentration in the supernatant subjected to the removal of the polymer was quantitatively determined, and from the determined concentration, the amount adsorbed to the molecular template polymer was determined. Table 2 and Figure 10 show the adsorption behavior results. The abscissa axis of Figure 10 indicates the concentration of cortisol (µM), and the ordinate axis indicates the bonded amount (µmol/g) determined from the calibration line as averaged over three times measurements. In the cases where the measurement results were 0 or less, almost the whole of cortisol was recovered in the supernatant liquid, and it is interpreted that the bonded amounts to MIP or NIP were small.

**[Table 2]**

| Concentration of cortisol (µM) | Bonded amount (µmol/g) | |
|---|---|---|
| | MIP | NIP |
| 0 | 0 | 0 |
| 125 | 0.55 | 0.44 |
| 250 | 0.54 | 0 or less (-0.15) |
| 500 | 2.61 | 0 or less (-0.1) |
| 1000 | 3.37 | 0 or less (-0.2) |

For NIP, cortisol was almost not adsorbed in a manner independent of the concentration, and in contrast to this, for MIP, the increase of the adsorbed amount was found with the increase of the concentration. From these results, it has been shown that the molecular template polymer prepared with the foregoing materials and method is able to specifically recognize cortisol. Consequently, a molecular template having a high recognition ability for a steroid hormone such as cortisol was able to be provided.

### (Example 2)

### • Methacryloylation of Cortisol

In foregoing Example 1, a description was made on an example of the synthesis of MIP utilizing the hydrogen bonding between a raw material for a molecular template, such as itaconic acid and cortisol, a template molecule. Next, a description is made on an example of the synthesis of MIP utilizing the covalent bond between a raw material and the template molecule. The MIP prepared in Example 2 is referred to as MIP1. First, upon the synthesis of MIP1, cortisol, a template molecule, was transformed according to the following procedure.

First, in nitrogen atmosphere, cortisol (2.5 mmol, 907 mg) was dissolved in dry THF (40 mL), and triethylamine (30 mmol, 4.2 ml) was added to the resulting solution and the solution was ice cooled. To this cooled solution, dry THF (40 mL) in which methacryloyl chloride (15 mmol, 1.5 ml) was dissolved was slowly dropwise added, and the solution was stirred at 0°C for 1 hour, and then at room temperature for 4 hours.

Successively, ethyl acetate was added to the reaction liquid, and the organic phase was washed with a saturated aqueous solution of sodium hydrogen carbonate, citric acid and an aqueous solution of sodium chloride by using a separating funnel. Subsequently, the organic phase was dried with sodium sulfate.

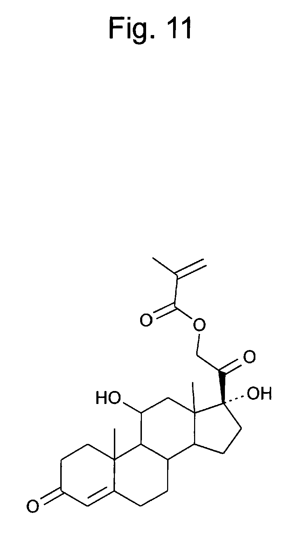

Next, the solvent was distilled off with an evaporator, and the extract was separated and purified with silica gel column chromatography (silica gel C-200, developing solvent: ethyl acetate/hexane = 1: 1) to yield a white solid (yield: 65%). Figure 11 shows the molecular structure of methacryloylated cortisol thus obtained.

Methacryloylated cortisol shown in Figure 11 was also able to be obtained by the following method. Specifically, in nitrogen atmosphere, in a two-neck flask, cortisol (2.5 mmol, 907 mg) and dimethylaminopyridine (0.25 mmol, 30.5 mg) were dissolved in dry THF (40 mL), and the resulting solution was ice cooled. Successively, triethylamine (30 mmol, 4.2 ml) and methacrylic anhydride (7.5 mmol, 1.2 ml) were slowly dropwise added to the solution, and the solution was stirred at 0°C for 1 hour, and then at room temperature for 2 days. Ethyl acetate was added to the reaction liquid, and the organic phase was washed with pure water three times by using a separating funnel, and dried with sodium sulfate. The solvent was distilled off with an evaporator, and the extract was separated and purified with silica gel column chromatography (silica gel C-200, developing solvent: ethyl acetate/hexane = 1:1) to yield a white solid (yield: 89%).

### • Synthesis of MIP1

By using as a template molecule the cortisol derivative, having a methacryloyl group introduced thereinto, prepared by either of the foregoing methods, MIP1 was synthesized according to the raw material composition shown in Table 3. A methacryloyl group has an ethylenically unsaturated group and is polymerization-reactive, and hence cortisol having a methacryloyl group introduced thereinto is copolymerizable with the added monomers shown in Table 3. Consequently, the raw material for the molecular template and the cortisol derivative can be strongly bonded to each other and recognize each other, and hence it is possible to produce a molecular template capable of capturing cortisol with a high selectivity.

Specifically, according to the recipe in Table 3, the polymerization of the molecular template polymer (MIP1) was performed. In a vial, 215.3 mg (0.5 mmol) of methacryloylated cortisol, 262 mg (2 mmol) of itaconic acid, 209 mg (2 mmol) of styrene and 1304 mg (10 mmol) of divinylbenzene (DVB) were added and dissolved in THF (10 ml); then the resulting solution was transferred into a φ18 x 180 mm test tube, and 2,2'-azobis(4-methoxy-2,4-dimethylvaleronitrile) (V-70) (0.28 mmol), a polymerization initiator, was dissolved in the solution. The test tube was sealed with a septum cap and the air in the test tube was replaced with nitrogen, and then a polymerization was performed in a water bath set at 30°C for 72 hours. The obtained polymer was vacuum-dried, ground with a mortar, then hydrolyzed with 50 ml of a 2M aqueous solution of sodium hydroxide/methanol = 1:1 for 48 hours, then washed with 1M hydrochloric acid/methanol = 1:1 and pure water/methanol = 1:1, and then subjected to a Soxhlet extraction for 6 hours with methanol. By performing hydrolysis, the cortisol derivative can be removed from MIP1.

**[Table 3]**

| Raw materials | MIP | NIP |
|---|---|---|
| Methacryloylated cortisol | 215.3 mg (0.5 mmol) | None |
| Itaconic acid | 262mg (2 mmol) | 262mg (2 mmol) |
| Styrene | 209 mg (4 mmol) | 209 mg (4 mmol) |
| DVB | 1304 mg (10 mmol) | 1304 mg (10 mmol) |
| V-70 | 86.4 mg (0.28 mmol) | 86.4 mg (0.28 mmol) |
| THF | 10 mL | 10 mL |

### • Adsorption Experiment of Cortisol

The polymer (MIP1) was weighed out in an amount of 5 mg, and 1 ml of each of the chloroform/hexane=4/1 solutions of cortisol regulated so as to respectively have the concentrations of 1000, 500, 250, 125 and 62.5 µmol/l was added to the weighed out MIP1; the resulting solutions were incubated at 25°C and 800 rpm for 48 hours. The solutions after the incubation were filtered with a syringe filter, and 0.5 ml of each of the resulting filtrates was sampled and vacuum-dried, and then redissolved in 0.5 ml of acetonitrile; the resulting acetonitrile solutions were subjected to a quantitative determination with LC. For the solutions containing no MIP1 added thereto, the same operations as described above were performed, and by using the resulting calibration line as the reference calibration line, the bonded amounts were determined. The membrane used for the filtration allows a solid of 50 µm or less to pass therethrough. From the results of the quantitative determination, the amounts of cortisol adsorbed to the molecular template polymer MIP1 in the respective concentrations were determined.

The HPLC conditions were as follows: For the column, CHROMOLITH (registered trademark) RP-18e 100-4.6 manufactured by Merck KGaA was used; for the eluent, acetonitrile/H₂O (70/30, volume/volume) was used; the flow rate was set at 1mL/min; and for detection, UV light (241 nm) was used.

### • Adsorption Experiment of Progesterone

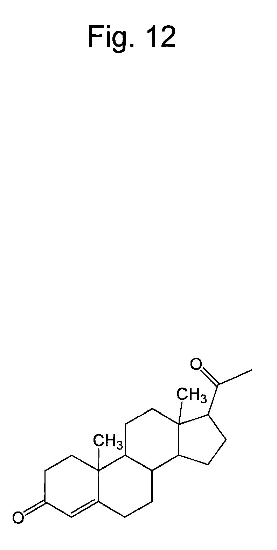

The polymer (MIP1) was weighed out in an amount of 5 mg, and 1 ml of each of the chloroform/hexane = 4/1 solutions of progesterone regulated to concentrations of 1000, 500, 250, 125 and 62.5 µmol/l was added to the weighed out MIP1; the resulting solutions were incubated at 25°C and 800 rpm for 48 hours. Figure 12 shows the chemical structure of progesterone. The solutions after the incubation were filtered with a syringe filter, and 0.5 ml of each of the resulting filtrates was sampled and vacuum-dried, and then redissolved in 0.5 ml of acetonitrile; the resulting acetonitrile solutions were subjected to a quantitative determination with LC. For the solutions containing no MIP1 added thereto, the same operations as described above were performed, and by using the resulting calibration line as the reference calibration line, the bonded amounts were determined. The membrane used for the filtration allows a solid of 50 µm or less to pass therethrough. From the results of the quantitative determination, the amounts of progesterone adsorbed to the molecular template polymer MIP1 in the respective concentrations were determined.

The HPLC conditions were as follows: for the column, CHROMOLITH (registered trademark) RP-18e 100-4.6 manufactured by Merck KGaA was used; for the eluent, acetonitrile/H₂O (70/30, volume/volume) was used; the flow rate was set at 1 mL/min; and for detection, UV light (241 nm) was used.

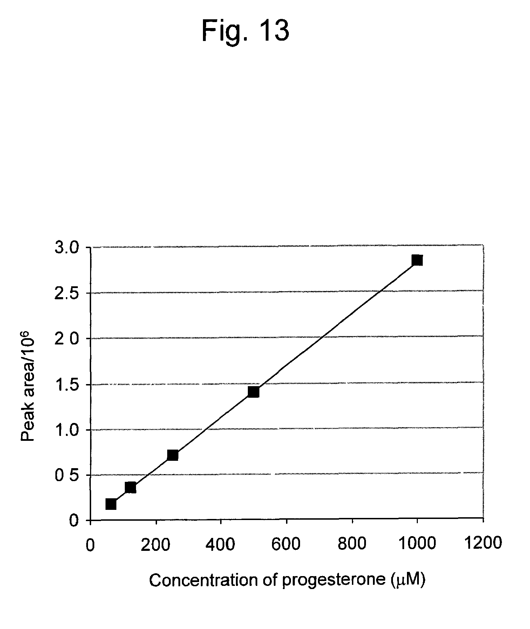

Before the adsorbed amount was determined, a HPLC calibration line was prepared for progesterone. Figure 13 shows the prepared calibration line. The abscissa axis of the graph indicates the concentration of progesterone (µM), and the ordinate axis indicates the peak area determined from HPLC. The samples respectively having the concentrations of progesterone of 62.5, 125, 250, 500 and 1000 µM were used, and the peak areas for the respective concentrations were plotted against the respective concentrations. The trendline of this plot was found to be represented by Y = 2818.7X, and the squared correlation coefficient (R²) was found to be 0.9999. By using this calibration line, the amounts adsorbed to MIP1 were derived. For the HLPC calibration line of cortisol, foregoing Figure 9 was utilized.

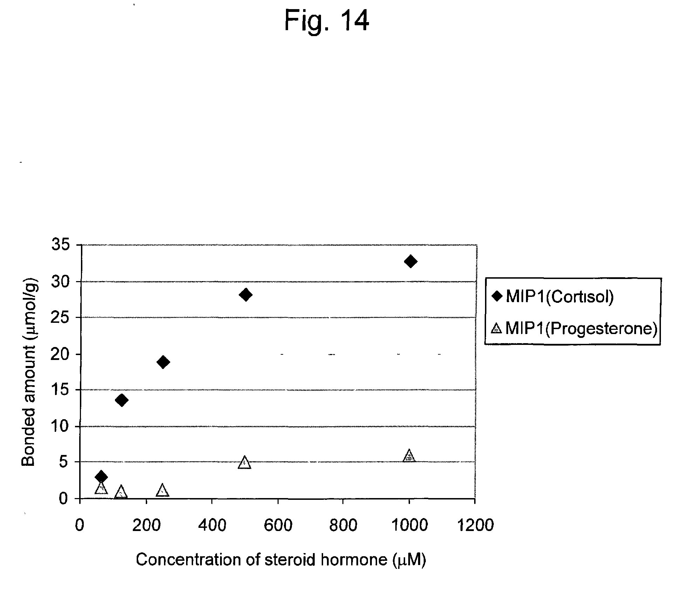

The concentrations in the supernatants subjected to the removal of the polymer were quantitatively determined, and on the basis of the results of the quantitative determination, the amounts adsorbed to the molecular template polymer MIP1 were determined. Table 4 and Figure 14 show the adsorption behavior results. The abscissa axis of Figure 14 indicates the concentration of a steroid hormone (cortisol or progesterone) (µM), and the ordinate axis indicates the bonded amount (µmol/g) determined from the calibration line as averaged over three times measurements.

As a result, when the concentration of the steroid hormone was 62.5 µM, the amount adsorbed to MIP1 of cortisol was 2.9 (µmol/g), and the amount adsorbed to MIP1 of progesterone was 1.5 (µmol/g); thus, a significant difference was obtained between these two adsorbed amounts. Accordingly, when these two types of steroid hormones are present in saliva, this MIP1 can capture cortisol with a higher selectivity. Similarly, when the concentration of the steroid hormone was 125 µM, the amount adsorbed to MIP1 of cortisol was 13.7 (µmol/g), and the amount adsorbed to MIP1 of progesterone was 1.0 (µmol/g); a significant difference was also found between these two adsorbed amounts. When the concentration of the steroid hormone was 250 µM, the amount adsorbed to MIP1 of cortisol was 19.0 (µmol/g), and the amount adsorbed to MIP1 of progesterone was 1.3 (µmol/g); a significant difference was also found between these two adsorbed amounts. When the concentration of the steroid hormone was 500 µM, the amount adsorbed to MIP1 of cortisol was 28.2 (µmol/g), and the amount adsorbed to MIP1 of progesterone was 5.2 (µmol/g); a significant difference was also found between these two adsorbed amounts. When the concentration of the steroid hormone was 1000 µM, the amount adsorbed to MIP1 of cortisol was 32.7 (µmol/g), and the amount adsorbed to MIP1 of progesterone was 6.0 (µmol/g); a significant difference was also found between these two adsorbed amounts.

**[Table 4]**

| Concentration of steroid hormone (µM) | Amount bonded to MIP1 (µmol/g) | |
|---|---|---|
| | Cortisol | Progesterone |
| 62.5 | 2.9 | 1.5 |
| 125 | 13.7 | 1.0 |
| 250 | 19.0 | 1.3 |
| 500 | 28.2 | 5.2 |
| 1000 | 32.7 | 6.0 |

As can be seen from a comparison between Table 2 and Table 4, for the same concentration of cortisol, as compared to the amount adsorbed to MIP in Example 1, the amount adsorbed to MIP1 in Example 2 was higher. In other words, in any concentration of cortisol, MIP1 was larger in the captured amount of cortisol as compared with MIP in Example 1. Therefore, for achieving a high sensitivity, it is more advantageous to methacryloylate cortisol, a template molecule, and to prepare the molecular template MIP1.

As presented above, the embodiments for implementing the present invention are described. Although MIP has such a selectivity and a capturing property as those of an antibody, a biopolymer, MIP is excellent in environmental tolerance and temperature resistance, because of being a non-natural synthetic substance. Accordingly, MIP has an advantage such that users can use MIP, for example, without being nervous about storing MIP. Consequently, it is possible to provide a chemical sensor easy to use for general consumers at home as well as medical personnel (medical doctors, medical technicians and nurses), assumed as users. In particular, by detecting with high sensitivity a steroid hormone such as cortisol closely related to stress disorder, it is possible to early diagnose the symptom of stress disorder, and to herewith contribute to the prevention and early treatment of stress disorder.

The present invention should not be limited to the foregoing embodiments and may include various modified examples. For example, the constitution of some embodiment may be partially replaced with another embodiment, and the constitution of some embodiment may include another embodiment as added thereto. Alternatively, the constitutions of the respective embodiments may be partially modified by adding or deleting other constitutions, or by replacing with other constitutions.

### Reference Signs List

- 1: Apparatus for detecting a chemical substance
- 10: Molecule capturing section
- 101: Capturing body
- 102: Support
- 103: Molecular template
- 104: Molecular template
- 11: Captured amount measurement section
- 111: Arrow
- 112: Arrow
- 14: Sample injection section
- 15: Sample conveyance section
- 16: Discharge section
- 17: Specimen
- 170: Target
- 171: Foreign substance A
- 172: Foreign substance B
- 20: Target
- 201: Monomer raw material A
- 202: Monomer raw material B
- 203: Monomer raw material C
- 21: Recognition site
- 22: Molecular template
- 501: Arrow
- 502: Arrow
- 80: Molecular template
- 82: Specimen
- 83: Labeled target
- 820: Target
- 821: Foreign substance A
- 822: Foreign substance B
- 832: Target moiety
- 831: Label moiety
- 84: Container
- 90: Capture/detection section
- 91: Sample injection section
- 92: Pretreatment layer
- 93: Specimen
- 930: Target
- 931: Foreign substance A
- 932: Foreign substance B

- 933: Arrow
The publications, patents and patent applications quoted in present Description are all incorporated in present Description by reference.

## Claims

1. A molecular template for a steroid hormone, comprising a polymer to interact with the steroid hormone.

2. The molecular template according to claim 1, wherein the polymer comprises two or more functional groups to interact with the steroid hormone in a polymerization unit.

3. The molecular template according to claim 2, comprising two or more carboxyl groups as the functional groups to interact with the steroid hormone in the polymerization unit.

4. The molecular template according to claim 3, wherein the steroid hormone is cortisol or a derivative of cortisol, and the polymer comprises itaconic acid as a polymerization unit.

5. A method for producing a molecular template, comprising:
performing the polymerization reaction of a monomer to interact with a steroid hormone in the presence of the steroid hormone; and
removing the steroid hormone from the polymer obtained by the polymerization reaction by washing the polymer.

6. The method for producing a molecular template according to claim 5, wherein the monomer comprises a monomer having two or more functional groups to interact with the steroid hormone.

7. The method for producing a molecular template according to claim 6, wherein the monomer comprises two or more carboxyl groups as the functional groups to interact with the steroid hormone.

8. The method for producing a molecular template according to claim 7, wherein the steroid hormone is cortisol or a derivative of cortisol, and the monomer is itaconic acid.

9. The method for producing a molecular template according to claim 5, wherein the monomer comprises two or more monomers each comprising a functional group to interact with the steroid hormone.

10. The method for producing a molecular template according to claim 5, wherein the steroid hormone comprises a functional group to undergo copolymerization reaction with the monomer.

11. The method for producing a molecular template according to claim 10, wherein the functional group is a methacryloyl group.

12. The method for producing a molecular template according to claim 10 or 11, wherein the monomer comprises a monomer comprising two or more functional groups to interact with the steroid hormone.

13. The method for producing a molecular template according to claim 12, wherein the monomer comprises two or more carboxyl groups as the functional groups to interact with the steroid hormone.

14. The method for producing a molecular template according to claim 10, wherein the steroid hormone is methacryloylated cortisol and the monomer is itaconic acid.

15. An apparatus for detecting a chemical substance comprising:
a molecule capturing section comprising a capturing body including the molecular template according to any one of claims 1 to 4; and
a captured amount measurement section to quantitatively determine the steroid hormone captured in the molecule capturing section.

16. The apparatus for detecting a chemical substance according to claim 15, wherein the molecule capturing section is constituted so as to enhance the sensitivity of detection of a steroid hormone by a competition method or a substitution method.

17. The apparatus for detecting a chemical substance according to claim 15 or 16, wherein a steroid hormone is quantitatively determined in the captured amount measurement section by using a surface plasmon resonance measurement method, a quartz crystal microbalance measurement method, an electrochemical impedance method, a colorimetric method or a fluorescence method.

18. A method for detecting a chemical substance, comprising:
capturing a steroid hormone in a molecule capturing section by bringing a specimen including the steroid hormone into contact with the molecule capturing section comprising a capturing body including the molecular template according to any one of claims 1 to 4; and
quantitatively determining the steroid hormone captured in the molecule capturing section.

19. The method for detecting a chemical substance according to claim 18, wherein the molecule capturing section is constituted so as to enhance the detection sensitivity of a steroid hormone by a competition method or a substitution method.
